# EUROPÄISCHE PATENTSCHRIFT

(11) **EP 1 150 683 B1**
(45) Veröffentlichungstag und Bekanntmachung des Hinweises auf die Patenterteilung: **08.10.2003**
(21) Anmeldenummer: 00906295.1
(22) Anmeldetag: 08.02.2000
(51) Int. Cl.: A61K 31/565, A61P 5/34

(54) **VERWENDUNG VON DIENOGEST IN HOHER DOSIERUNG**
UTILIZATION OF DIENOGEST IN HIGH DOSES
UTILISATION DE DIENOGEST EN DOSE ELEVEE

(30) Priorität: 18.02.1999 DE 19908762
(43) Veröffentlichungstag der Anmeldung: 07.11.2001
(73) Patentinhaber: Schering AG, 13353 Berlin (DE)
(72) Erfinder: OETTEL, Michael, D-07743 Jena (DE); MOORE, Claudia, D-07751 Drackendorf (DE); SCHINDLER, Adolf, Eduard, D-45257 Essen (DE); CHRISTENSEN, Bernd, D-45145 Essen (DE); MÜLLER, Andreas, D-07646 Laasdorf (DE)
(74) Vertreter: Cramer, Eva-Maria
(86) Internationale Anmeldenummer: EP0000982
(87) Internationale Veröffentlichungsnummer: WO00048604

(56) Entgegenhaltungen:
- DE-A- 3 238 984
- OETTEL M ET AL: "A 19-NORPROGESTIN WITHOUT 17ALPHA-ETHINYL GROUP II: DIENOGEST FROM A PHARMACODYNAMIC POINT OF VIEW" DRUGS OF TODAY / MEDICAMENTOS DE ACTUALIDAD,ES,J.R. PROUS SS.A. INTERNATIONAL PUBLISHERS, Bd. 31, Nr. 7, 1. Oktober 1995 (1995-10-01), XP002046623 ISSN: 0025-7656
- PASQUALINI JR ET AL: "Progestins: Present and Future" J STEROID BIOCHEM MOLEC BIOL, Bd. 59, Nr. 5/6, 1996, Seiten 357-363, XP000909721
- FOSTER RH AND WILDE MI: "Dienogest" DRUGS, Bd. 56, November 1998 (1998-11), Seiten 825-833, XP000909612

## Beschreibung

Die vorliegende Erfindung betrifft die Verwendung von Dienogest in hoher Dosierung zur Reduktion des Brustdrüsenkörpers.

Durch Mastopathien verursachte Vergrößerungen des Brustdrüsenkörpers treten sehr häufig bei Frauen in der Geschlechtsreife und insbesondere im Alter zwischen 35 und 50 Jahren auf. Je nach Form der Mastopathie, einfache, gering proliferierende oder atypisch proliferierende Mastopathie, gehen derartige Veränderungen, auch mit einer Erhöhung des Risikos an einem Mammakarzinom zu erkranken, einher. Neben diesem erhöhten Krebsrisiko treten auch andere Symptome auf, wie knotige Verhärtungen, Schmerzen, Sekretionen. Charakteristisch ist hierbei die prämenstruelle Verstärkung dieser Symptome.

Eine Therapie der Mastopathien ist meist nicht erforderlich, da die Brust an sich gesund, jedoch nur mitunter erheblich vergrößert ist.

Im Stand der Technik werden Hormonbehandlungen beschrieben bei denen verschiedene Gestagene allein oder in Kombination mit Estrogenen zur Prophylaxe oder zur Therapie des Brustkrebses der Frau verwendet werden.

In der DE 40 19 670 A1 ist ein Arzneimittel zur Behandlung der Wechseljahre der Frau beschrieben. Dieses ist ein Estrogen-Gestagengemisch mit dem Progesteronabkömmling Chlormadinonacetat als Gestagengehalt.

Die DE 197 05 229 A1 offenbart Mittel zur hormonalen Kontrazeption mit drei Hormonkomponenten, welche auch zur Behandlung und/oder Prophylaxe von Tumoren der Brustdrüsen geeignet sind. Das Mittel umfaßt eine erste Hormonkomponente, welche mindestens ein synthetisches Estrogen umfaßt, eine zweite Hormonkomponente, welche mindestens ein biogenes Estrogen umfaßt und eine dritte Hormonkomponente, welche mindestens ein Gestagen zur kontinuierlichen und kombinierten Verabreichung umfaßt.

WO 9010462 beschreibt eine Kombinationstherapie zur Behandlung von estrogenempfindlichen Erkrankungen. Hierbei wird bei Brust- oder Endometriumkrebs die Behandlung mit einem Antiestrogen und wenigstens einer weiteren Komponente aus der Gruppe von Androgenen, Progestinen, Inhibitoren für die Bildung von Sexualhormonen, Inhibitoren der Prolaktinsekretion, Inhibitoren der Wachstumshormonsekretion und Inhibitoren der ACTH-Sekretion, beschrieben.

In der EP 0 654 267 A1 wird die Verwendung von Dienogest als Carcinostatikum für die Hormontherapie beschrieben.

Es wird hierbei die Behandlung oder Prophylaxe von Gebärmutter- und/oder Brustkrebsen beschrieben.

Die bekannten Hormontherapien zur Behandlung von Brustkrebsen weisen eine Reihe von Nachteilen auf. So ist es beispielsweise nicht möglich, den gesunden Brustkörper zu reduzieren oder auch eine Prophylaxe von Mastopathien durchzuführen. Aufgabe der vorliegenden Erfindung ist es daher, eine neue Hormontherapie zur Verfügung zu stellen, welche diese Nachteile überwindet.

Die Aufgabe wird erfindungsgemäß durch die Verwendung von Dienogest in hoher Dosierung zur Reduktion des Brustdrüsenkörpers gelöst.

Die Aufgabe wird ferner durch Verwendung von Dienogest in hoher Dosierung zur Herstellung von pharmazeutischen Zusammensetzungen zur Reduktion des Brustdrüsenkörpers gelöst.

Erlindungsgemäß bevorzugt ist die Verwendung von Dienogest, wobei die Dosierung das mindestens 10fache der Ovulationsdosis ist.

Besonders bevorzugt ist dabei die Verwendung von Dienogest, wobei die Dosierung mindestens 1mal täglich mindestens 10 mg Dienogest beträgt.

Insbesondere bevorzugt ist die Verwendung von Dienogest, wobei die Dosierung mindestens drei Monate lang andauert.

Erfindungsgemäß ist ferner die Verwendung von Dienogest unter oraler, transdermaler, vaginaler, subkutaner, intramuskulärer oder intravenöser Applikation.

Dienogest (17-Hydroxy-3-oxo-19-nor-17α-pregna-4,9-dien-21-nitril, Dienogestril) ist ein Gestagen und ein synthetisches Derivat des Hydroxyprogesterons. Dienogest wird in vielen Kontrazeptiva als Gestagenkomponente verwendet.

Überraschenderweise wurde gefunden, daß eine erhöhte Dosis an Dienogest, welche ein Vielfaches über der für die Hemmung der Ovulation erforderlichen liegt, in der Lage ist, den Brustdrüsenkörper zu reduzieren. Es wurde ferner gefunden, daß auch Rückbildungen mastopathischer Veränderungen des Brustkörpers bewirkt werden. Daneben wurde ferner gefunden, daß Dienogest auch zur Prophylaxe von Mastopathien hervorragend geeignet ist. Dienogest in hohen Dosierungen beugt der Bildung von mastopathischen Veränderungen des Brustkörpers vor.

Überraschenderweise wurde ferner gefunden, daß durch hohe Gaben von Dienogest die Blutfettwerte in vorteilhafter Weise beeinflußt werden. Der HDL-Cholesterolspiegel wird unter der erfindungsgemäßen Verwendung von Dienogest erhöht. Dies führt zu einem positiven Einfluß auf das kardiovaskuläre System, das Risiko für koronare Erkrankungen wird verringert.

Ein weiterer Gegenstand der vorliegenden Erfindung sind pharmazeutische Zusammensetzungen zur Reduktion des Brustdrüsenkörpers enthaltend Dienogest in hoher Dosierung zusammen mit pharmazeutisch annehmbaren Hilfs-, Träger- und/oder Zusatzstoffen.

Gegenstand der vorliegenden Erfindung sind auch Arzneimittel zur oralen, transdermalen, vaginalen, subcutanen, intravenösen oder intramuskulären Applikation, die neben üblichen Träger- und Verdünnungsmitteln Dienogest als Wirkstoff enthalten.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien, insbesondere Vaginalsuppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Transdermale Applikationsformen können beispielsweise aus wirkstoffhaltigen Pflastern bestehen. Derartige Systeme sind bekannt.

Geeignete Suppositorien, insbesondere zur vaginalen Applikation lassen sich beispielsweise durch Vermischen mit dafür vorgesehenen Trägermitteln wie Neutralfetten oder Polyäthylenglykol bzw. deren-Derivaten herstellen.

### Beispiel

In einer klinischen Studie wurde an 21 Patientinnen im Alter von 18 bis 52 Jahren die Reduktion des Brustdrüsenkörpers sonographisch nachgewiesen.

Über einen Zeitraum von 24 Wochen wurden im Abstand von 12 Stunden jeweils 10 mg Dienogest als Tablett oral verabreicht. Die Tagesdosis betrug somit 20 mg Dienogest. Die Untersuchung der Brust erfolgte sonographisch vor der Behandlung sowie nach 12 und 24 Wochen kontinuierlicher Therapie.

Bei allen Frauen verkleinerte sich der Brustdrüsenkörper im Mittel signifikant. Anzeichen mastopathischer Veränderungen wie Duktektasien bildeten sich vollständig zurück.

## Patentansprüche

1. Verwendung von Dienogest in einer Dosierung nur mindestens 10 mg, zur Herstellung von pharmazeutischen Zusammensetzungen zur Reduktion des Brustdrüsenkörpers.

2. Pharmazeutische Zusammensetzung zur Reduktion des Brustdrüsenkörpers enthaltend Dienogest in einer Dosierung, nur mindestens 10 mg, zusammen mit pharmazeutisch annehmbaren Hilfs-, Träger- und/oder Zusatzstoffen.

3. Pharmazeutische Packung enthaltend Dienogest in mehreren Tagesdosiseinheiten,
**dadurch gekennzeichnet,**
**daß** die Dosierung unter oraler, transdermaler, vaginaler, subkutaner, intramuskulärer oder intravenöser Applikation mindestens 1mal täglich 10 mg Dienogest beträgt.

4. Pharmazeutische Packung nach Anspruch 3,
**dadurch gekennzeichnet,**
**daß** die Dosierung mindestens 3 Monate lang andauert.

## Claims

1. Use of dienogest in a dose of at least 10 mg for the production of pharmaceutical compositions for the reduction of the mammary corpus.

2. Pharmaceutical composition for the reduction of the mammary corpus comprising dienogest in a dose of at least 10 mg, together with pharmaceutically acceptable excipients, vehicles and/or additives.

3. Pharmaceutical pack comprising dienogest in a number of daily dose units,
**characterized in that**
the dose under oral, transdermal, vaginal, subcutaneous, intramuscular or intravenous administration at least once daily is 10 mg of dienogest.

4. Pharmaceutical pack according to Claim 3,
**characterized in that**
the dose lasts for at least 3 months.

## Revendications

1. Utilisation du diénogest en un dosage d'au moins 10 mg pour la production de compositions pharmaceutiques destinées à la réduction du corps mammaire.

2. Composition pharmaceutique destinée à la réduction du corps mammaire, comprenant du diénogest en un dosage d'au moins 10 mg, conjointement avec des excipients, des véhicules et/ou des additifs pharmaceutiquement acceptables.

3. Emballage pharmaceutique comprenant du diénogest en plusieurs doses unitaires journalières, **caractérisé en ce que** le dosage à administrer au moins une fois par jour par voie orale, transdermique, vaginale, sous-cutanée, intramusculaire ou intraveineuse comprend 10 mg de diénogest.

4. Emballage pharmaceutique selon la revendication 3, **caractérisé en ce que** le dosage dure au moins 3 mois.
